# EUROPÄISCHE PATENTSCHRIFT

(11) **EP 1 642 121 B1**
(45) Veröffentlichungstag und Bekanntmachung des Hinweises auf die Patenterteilung: **07.03.2007**
(21) Anmeldenummer: 04740451.2
(22) Anmeldetag: 30.06.2004
(51) Int. Cl.: G01N 33/00, G01N 27/02

(54) **DRIFTKOMPENSATION FÜR EINEN IMPEDIMETRISCHEN ABGASSENSOR DURCH ANLEGEN EINER EINSTELLBAREN VORSPANNUNG**
DRIFT COMPENSATION FOR AN IMPEDIMETRIC EXHAUST GAS SENSOR BY VARIABLE BIAS VOLTAGE
COMPENSATION DE GLISSEMENT POUR CAPTEUR DE GAZ D'ECHAPPEMENT IMPEDIMETRIQUE, PAR APPLICATION D'UNE TENSION PRELIMINAIRE REGLABLE

(30) Priorität: 07.07.2003 DE 10330742
(43) Veröffentlichungstag der Anmeldung: 05.04.2006
(73) Patentinhaber: DaimlerChrysler AG, 70567 Stuttgart (DE)
(72) Erfinder: BIRKHOFER, Thomas, 88090 Immenstaad (DE); KNEZEVIC, Aleksandar, 88045 Friedrichshafen (DE); MÜLLER, Ralf, 88693 Deggenhausertal (DE); PLOG, Carsten, 88677 Markdorf (DE)
(86) Internationale Anmeldenummer: PCT/EP2004/007068
(87) Internationale Veröffentlichungsnummer: WO 2005/003755

(56) Entgegenhaltungen:
- DE-A- 10 011 562
- DE-C- 3 915 563
- LECHUGA L M ET AL: "THE AMMONIA SENSITIVITY OF PT/GAAS SCHOTTKY BARRIER DIODES" JOURNAL OF APPLIED PHYSICS, AMERICAN INSTITUTE OF PHYSICS. NEW YORK, US, Bd. 70, Nr. 6, 15. September 1991 (1991-09-15), Seiten 3348-3354, XP000263541 ISSN: 0021-8979
- STEIMER G ET AL: "Current-voltage characteristics of thin film SnO2 gas sensors: electronic artifacts and gas response" SENSORS AND MATERIALS MYU JAPAN, Bd. 9, Nr. 2, 1997, Seiten 107-116, XP008036596 ISSN: 0914-4935
- HIKITA K ET AL: "CO GAS IDENTIFICATION BY USE OF COMPLEX IMPEDANCE OF PN HETEROCONTACT COMPRISING OF NA-ADDED CUO AND ZNO" JOURNAL OF THE CERAMIC SOCIETY OF JAPAN, XX, XX, Bd. 102, Nr. 9, September 1994 (1994-09), Seiten 810-817, XP008036427 ISSN: 0914-5400

## Beschreibung

Die Erfindung betrifft einen Gassensor zur Detektion einer Gaskomponente im Abgas einer Brennkraftmaschine mit einer Steuer- und Auswerteeinheit mit den Merkmalen des Oberbegriffs des Anspruchs 1 und ein Verfahren zum Betreiben eines Gassensors mit den Merkmalen des Oberbegriffs des Anspruchs 9.

Aus der Europäischen Patenschrift EP 0 426 989 Bl sind ein Gassensor und ein Betriebsverfahren für einen Gassensor bekannt. Der Gassensor weist eine als Kondensator wirkende Elektrodenstruktur mit zwei Anschlüssen auf. Die Elektrodenstruktur ist mit einer sensitiven Schicht überzogen. Diese Schicht ist sensitiv bezüglich einer zu messenden Gaskomponente, wobei als Messgröße die sich mit der Konzentration der Gaskomponente ändernde Kapazität an den Anschlüssen abgreifbar ist. Allerdings ist die Langzeitstabilität eines solchen Sensors vielfach unbefriedigend, was insbesondere für seinen Einsatz in Brennkraftmaschinenabgasen nachteilig ist.

Aufgabe der Erfindung ist es daher, einen Abgassensor zur Detektion einer Gaskomponente im Abgas einer Brennkraftmaschine und ein Betriebsverfahren für einen Abgassensor anzugeben, bei welchen eine hohe Langzeitstabilität erreicht wird.

Die Aufgabe wird erfindungsgemäß durch einen Gassensor mit den Merkmalen des Anspruchs 1 und durch ein Verfahren mit den Merkmalen des Anspruchs 9 gelöst.

Der erfindungsgemäße Abgassensor ist dadurch gekennzeichnet, dass eine dem Abgassensor zugeordnete Steuer- und Auswerteeinheit eine Vorspannung an den ersten und/oder an den zweiten Anschluss der Elektrodenstruktur der Sensoreinheit anlegt, wobei die Höhe der Vorspannung in Abhängigkeit von einer Sensoreigenschaft und/oder in Abhängigkeit von einer Sensorbelastung einstellbar ist.

Die durch teilweise hohe Temperaturen und die Einwirkung aggressiver Gase gekennzeichneten Einsatzbedingungen im Abgas einer Brennkraftmaschine stellen eine mit zunehmender Dauer zunehmende Belastung des Abgassensors dar und können eine Änderung von wichtigen Sensoreigenschaften bewirken. Daraus kann eine Alterung des Abgassensors resultieren, die sich insbesondere ungünstig auf die Langzeitstabilität der von der Sensoreinheit bereitgestellten Messgröße auswirkt. Dabei wird unter der Messgröße eine von den Umgebungsbedingungen abhängige Eigenschaft der Sensoreinheit verstanden. Dies ist dies die komplexe Impedanz der Sensoreinheit bzw. eine daraus abgeleitete Größe. Betroffen von Änderungen sind hauptsächlich die Stabilität des Nullpunktssignals und die Empfindlichkeit des Abgassensors bezüglich der zu detektierenden Gaskomponente. Die Sensorbelastung kann dabei beispielsweise durch die Betriebsdauer quantifiziert werden.

Durch das erfindungsgemäße Anlegen einer Vorspannung an die Anschlüsse der Sensoreinheit werden die Sensoreigenschaften stabilisiert, bzw. Auswirkungen der Sensorbelastung oder die alterungsbedingte Verschlechterung dieser Sensoreigenschaften kompensiert. Bei der Vorspannung kann es sich dabei um eine zusätzlich zur Betriebsspannung angelegte Offsetspannung oder um eine die Betriebsspannung korrigierende Spannung handeln. Vorzugsweise ist die Vorspannung in Abhängigkeit von der Abweichung einer Sensoreigenschaft von einem vorgegebenen oder vorgebbaren Sollwert einstellbar. Vorzugsweise wird durch diese einstellbare Vorspannung die Messgröße justiert, so dass Fehlmessungen auch nach einer langen Betriebsdauer und hohen Sensorbelastung vermieden werden.

Bei der Messgröße handelt es sich um eine zwischen den Anschlüssen der Sensoreinheit komplexe Impedanz oder eine daraus abgeleitete Eigenschaft der Sensoreinheit. Diese wird von der Steuer- und Auswerteeinheit ausgewertet und danach die einzustellende Vorspannung ermittelt und an einen Anschluss oder an die Anschlüsse der Sensoreinheit angelegt. Gegebenenfalls werden dabei weitere Signale, die vorzugsweise ebenfalls vom Gassensor bereitgestellt werden oder am Abgassensor abgegriffen werden ebenfalls ausgewertet, um die Vorspannung entsprechend einstellen und damit beispielsweise eine Degradation des Gassensors kompensieren zu können.

In Ausgestaltung der Erfindung ist die Höhe der Vorspannung in Abhängigkeit von einem Referenzwert der Messgröße einstellbar. Vorzugsweise handelt es sich bei dem Referenzwert der Messgröße um einen Wert der Messgröße bei einer vorgebbaren Konzentration der zu detektierenden Gaskomponente oder einer anderen Gaskomponente, gegenüber der ebenfalls eine Sensitivität gegeben ist. Im einfachsten Fall kann der Wert der Messgröße bei Abwesenheit der zu detektierenden Gaskomponente als Referenzwert dienen. Der Wert der Messgröße bei definierten Messbedingungen, wie beispielsweise eine bekannte Abgaszusammensetzung oder ein definierter Betriebszustand der Brennkraftmaschine, kann ebenfalls als Referenzwert dienen.

In weiterer Ausgestaltung der Erfindung ist die Höhe der Vorspannung in Abhängigkeit von der Empfindlichkeit der Sensoreinheit einstellbar. Unter Empfindlichkeit ist hierbei die auf einen Konzentrationsunterschied einer Gaskomponente bezogene Differenz zweier zugehöriger Werte der Messgröße zu verstehen.

In weiterer Ausgestaltung der Erfindung ist die Höhe der Vorspannung in Abhängigkeit von einer zwischen der Elektrodenstruktur der Sensoreinheit und einem Schaltkreis des Abgassensors messbaren elektrischen Bezugsgröße einstellbar. Bei dem Schaltkreis kann es sich dabei beispielsweise um eine im Abgassensor integrierte elektrische Heizung oder einen Mess-Schaltkreis zur Messung einer weiteren Größe handeln. Derartige Schaltkreise sind üblicherweise elektrisch isoliert von der Sensoreinheit im Abgassensor integriert und über elektrische Bezugsgrößen, wie beispielsweise die Induktivität, Kapazität oder Leitfähigkeit mit der Sensoreinheit gekoppelt und können den Wert der Messgröße beeinflussen und ebenfalls einer alterungsbedingten Änderung unterliegen. Die elektrischen Bezugsgrößen werden von der Steuer- und Auswerteeinheit ermittelt und ausgewertet. Mit dem Anlegen einer in Abhängigkeit von diesen Bezugsgrößen einstellbaren Vorspannung an einen Anschluss oder die Anschlüsse der Sensoreinheit werden die Querbeeinflussungen bzw. deren Änderungen kompensiert.

In weiterer Ausgestaltung weist der Gassensor einen mit einer Isolierschicht abgedeckten Schaltkreis zur Temperaturmessung auf, wobei die Sensoreinheit auf der Isolierschicht aufgebracht ist und die Höhe der Vorspannung in Abhängigkeit von einer zwischen der Elektrodenstruktur der Sensoreinheit und dem Schaltkreis zur Temperaturmessung messbaren elektrischen Bezugsgröße einstellbar ist. Vorzugsweise ist die Vorspannung in Abhängigkeit von der elektrischen Leitfähigkeit der Isolierschicht einstellbar. Diese kann einer Änderung, beispielsweise durch Eindiffundieren von Fremdatomen, unterliegen, wodurch beim Betrieb des Schaltkreises zur Temperaturmessung eine mit der Betriebszeit oder Sensorbelastung veränderliche Beeinflussung der Messgröße resultieren kann. Durch die beispielsweise in Abhängigkeit der Leitfähigkeit zwischen der Elektrodenstruktur der Sensoreinheit und dem Schaltkreis zur Temperaturmessung abhängig einstellbaren Vorspannung kann diesen Einflüssen begegnet werden.

In weiterer Ausgestaltung der Erfindung ist die Höhe der Vorspannung in Abhängigkeit von der Betriebsdauer des Gassensors einstellbar. Dabei kann es vorteilhaft sein, die Betriebsbedingungen, wie die Abgastemperatur oder die Konzentration bestimmter Abgaskomponenten, zur Gewichtung heranzuziehen.

In weiterer Ausgestaltung der Erfindung weist die Vorspannung eine positive Polung in Bezug auf eine Betriebsspannung eines Schaltkreises des Abgassensors auf. Der Betrieb einer im Abgassensor integrierten elektrischen Heizung oder eines Mess-Schaltkreises kann je nach Ausführung des Abgassensors bzw. des Schaltkreises einen mehr oder weniger starken Einfluss auf die Empfindlichkeit des Abgassensors oder die Größe der Messwerte ausüben. Durch eine positive Vorpolung gegenüber einem solchen Schaltkreis können geänderte oder alterungsbedingt eingetretene Verschlechterungen der Abgassensoreigenschaften wirksam kompensiert werden. Dabei ist die Polung der Vorspannung vorzugsweise positiv in Relation zu dem höchsten Potential des betreffenden Schaltkreises.

In weiterer Ausgestaltung der Erfindung ist der Abgassensor zur Erfassung der Gaskomponente Ammoniak ausgelegt. Hierzu umfasst die dem Abgas ausgesetzte Sensoreinheit vorzugsweise eine als planare Interdigitalkondensatorstruktur mit in der Art zweier Kämme mit ineinandergreifenden Elektroden ausgeführte Elektrodenstruktur und eine darauf aufgebrachte Funktionsschicht, deren elektrische Leitfähigkeit und/oder Dielektrizitätskonstante von der Ammoniakkonzentration des Abgases abhängig ist. Von der Steuer- und Auswerteeinheit wird die Impedanz zwischen den Anschlüssen der Sensoreinheit ermittelt und so die Konzentration der Gaskomponente Ammoniak im Abgas bestimmt.

Das erfindungsgemäße Verfahren zum Betreiben eines Abgassensors ist dadurch gekennzeichnet, dass eine gegenüber einem Massepotential definierte Vorspannung an den ersten und/oder an den zweiten Anschluss der Elektrodenstruktur der gassensitiven Sensoreinheit angelegt wird, wobei die Höhe der Vorspannung in Abhängigkeit von einer Sensoreigenschaft und/oder in Abhängigkeit von einer Sensorbelastung eingestellt wird. Dabei wird vorzugsweise von einer dem Abgassensor zugeordneten Steuer- und Auswerteeinheit die entsprechende Sensoreigenschaft und/oder eine mit der Sensorbelastung korrelierende Größe erfasst und die Vorspannung entsprechend eines vorgegebenen Zusammenhangs eingestellt. Ebenfalls vorteilhaft ist eine iterativ vorgenommene Einstellung der Vorspannung auf der Basis dieser Größe, um eine Veränderung der Sensoreigenschaften so weit als möglich zu kompensieren. Durch dieses Verfahren wird ein sich selbst stabilisierender Abgassensor erhalten.

In Ausgestaltung des Verfahrens wird die Höhe der Vorspannung in Abhängigkeit von der Nullpunktsdrift der elektrischen Messgröße eingestellt. Durch diese Maßnahme kann sowohl einem Wegdriften des Nullpunktswerts der Messgröße als auch einer Empfindlichkeitsdrift des Abgassensors im Laufe der Betriebsdauer entgegengewirkt werden.

In weiterer Ausgestaltung des Verfahrens wird die Höhe der Vorspannung in Abhängigkeit von einer Empfindlichkeitsdrift des Abgassensors eingestellt. Vorzugsweise wird von einer dem Abgassensor zugeordneten Steuer- und Auswerteeinheit die Empfindlichkeit des Abgassensors von Zeit zu Zeit ermittelt und die Vorspannung entsprechend eines funktionalen Zusammenhangs oder iterativ so geändert, dass die Empfindlichkeitsdrift so weit als möglich kompensiert wird. Hierzu kann die Empfindlichkeit gegenüber der eigentlich zu erfassenden Abgaskomponente oder alternativ eine gegenüber einer anderen Abgaskomponente vorhande Querempfindlichkeit herangezogen werden.

In weiterer Ausgestaltung des Verfahrens wird die Höhe der Vorspannung zu vorgebbaren Zeitpunkten eingestellt. Die Zeitpunkte können sich beispielsweise an der Betriebsdauer orientieren. Vorteilhaft ist die Neuermittlung bzw. Neueinstellung der Vorspannung in gleichmäßigen Zeitintervallen des Sensorbetriebs, etwa alle 10 bis 100 Stunden.

In weiterer Ausgestaltung des Verfahrens wird die Höhe der Vorspannung bei jedem n-ten Einschaltvorgang des Abgassensors eingestellt. Besonders vorteilhaft ist die Neuermittlung bzw. Neueinstellung der Vorspannung bei jedem Einschaltvorgang des Abgassensors. Damit wird die Zuverlässigkeit des Abgassensors bei jeder erneuten Inbetriebnahme sichergestellt.

In weiterer Ausgestaltung des Verfahrens wird die Vorspannung positiv in Bezug auf eine Betriebsspannung eines elektrisch von der Sensoreinheit isolierten Schaltkreises des Abgassensors eingestellt. Im Falle eines planar aufgebauten Abgassensors ist es besonders vorteilhaft, die Vorspannung positiv in Bezug auf eine Betriebsspannung eines unterhalb und isoliert von der Sensoreinheit als oberster Schicht angeordneten Schaltkreises einzustellen.

Im Folgenden wird die Erfindung anhand von Zeichnungen und zugehörigen Beispielen näher erläutert. Dabei zeigen:
- Fig. 1: eine schematisch als Explosionszeichnung dargestellte bevorzugte Ausführungsform des erfindungsgemäßen Abgassensors und
- Fig. 2: ein Diagramm zur Verdeutlichung einer typischen Veränderung von Abgassensoreigenschaften.

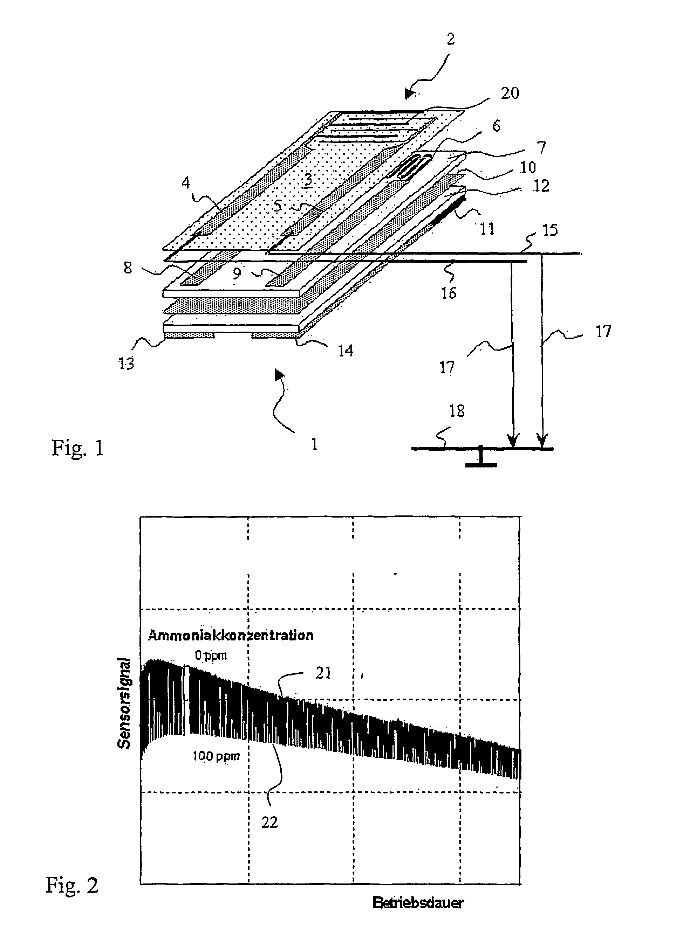

Nachfolgend wird eine bevorzugte Ausführungsform des erfindungsgemäßen, in Planartechnik ausgeführten Abgassensors anhand der Fig. 1 erläutert. Der Abgassensor 1 ist auf einem ersten, vorzugsweise aus Aluminiumoxidkeramik gebildeten Substrat 12 aufgebaut.

Auf der Unterseite des ersten Substrats 12 ist eine Heizerstruktur 11 mit zwei zugehörigen Anschlüssen 13, 14 zum Anschluss einer Heizspannung aufgebracht. Vorzugsweise sind die Heizerstruktur 11 und die Anschlüsse 13, 14 in Dickschichttechnik, alternativ auch in Dünnschichttechnik gefertigt. Auf dem ersten Substrat 12 ist ein ebenfalls vorzugsweise aus Aluminiumoxidkeramik gebildetes zweites Substrat 7 angeordnet, wobei es vorteilhaft ist, zwischen dem ersten Substrat 12 und dem zweiten Substrat 7, wie in Fig. 1 gezeigt, eine vorzugsweise geschlossene Trennschicht 10 aus einem elektrisch leitfähigen Material vorzusehen. Dabei kann ein nicht dargestellter Anschluss zum Anlegen einer Betriebsspannung an die Trennschicht 10 vorgesehen sein.

Auf dem zweiten Substrat 7 ist ein, vorzugsweise ebenfalls schichtartig ausgeführter Temperaturfühler 6 mit zwei Anschlüssen 8, 9 aufgebracht. Dabei ist es vorteilhaft, diesen beispielsweise als planares Widerstandsthermometer ausgebildeten Temperaturfühler 6 über der Heizerstruktur 11 anzuordnen.

Eine elektrisch isolierend wirkende Isolierschicht 3 deckt den Temperaturfühler 6 und die Anschlüsse 8, 9 ab. Auf der Isolierschicht 3 ist, vorzugsweise ebenfalls mittels einer Schichttechnologie, eine bevorzugt als Interdigitalstruktur mit kammartig ineinandergreifenden Leiterbahnen ausgeführte Elektrodenstruktur 20 mit einem ersten Anschluss 4 und einem zweiten Anschluss 5 aufgebracht. Typischerweise liegt die Breite der Leiterbahnen und deren Abstand im Bereich zwischen 1 µm und 100 µm.

Die Elektrodenstruktur 20 ist mit einer hier nicht dargestellten Funktionsschicht beschichtet, welche die Sensitivität des Abgassensors 1 maßgeblich bestimmt. Vorzugsweise ist die Funktionsschicht aus einem Zeolith gebildet, dessen Zusammensetzung und Porosität auf die zu messende Gaskomponente abgestimmt ist. Obgleich die Dicke der Funktionsschicht bis zu einigen Zehntel mm betragen kann, ist eine Dicke im Bereich von etwa 1 bis 50 µm bevorzugt. Die Elektrodenstruktur 20 mit der nicht dargestellten Funktionsschichtabdeckung bilden die Sensoreinheit 2 des Abgassensors 1. Vorzugsweise ist die Sensoreinheit 2, getrennt durch die Isolierschicht 3, direkt über dem Temperaturfühler 6 des Abgassensors 1 angeordnet. Auf diese Weise kann die Temperatur der Sensoreinheit 2 besonders genau erfasst und durch Bestromung der Heizerstruktur 11 eingeregelt werden.

Eine erste Versorgungsleitung 15 und eine zweite Versorgungsleitung 16 sind zu einer nicht dargestellten Steuer- und Auswerteeinheit geführt, welche die für den Betrieb des Abgassensors 1 notwendigen Betriebsspannungen an die dafür vorgesehenen Anschlüsse liefert und die Auswertung der zwischen dem ersten Anschluss 4 und dem zweiten Anschluss 5 der Elektrodenstruktur 20 vorliegenden elektrischen Messgröße übernimmt. Von der Steuer- und Auswerteeinheit wird eine gegenüber einem Massepotential 18 definierte Vorspannung 17 an den ersten Anschluss 4 und/oder an den zweiten Anschluss 5 der Sensoreinheit 2 gelegt, was weiter unten näher erläutert wird.

Nachfolgend werden das Funktionsprinzip und der normale Betrieb des Abgassensors 1 erläutert. Dabei wird davon ausgegangen, dass die Sensoreinheit 2 dem Abgas einer nicht dargestellten Otto/Diesel-Brennkraftmaschine ausgesetzt ist. Zum Betrieb des Abgassensors 1 wird dieser zunächst aufgeheizt. Hierzu wird von der Steuer- und Auswerteeinheit eine Heizspannung an die Anschlüsse 13, 14 gelegt. Dabei erfolgt eine Einregelung auf die vorgebbare Betriebstemperatur von etwa 300° C bis 800° C mit Hilfe des ebenfalls an die Steuer- und Auswerteeinheit angeschlossenen Temperaturfühlers 6. Von der Steuer- und Auswerteeinheit wird dann eine Betriebsspannung an die Anschlüsse 4, 5 der Sensoreinheit 2 angelegt. Diese Betriebsspannung ist eine Wechselspannung mit einer vorgegebenen Frequenz, die typischerweise im Bereich zwischen 10² Hz bis 10⁵ Hz liegt. Als Messgröße dient die komplexe Impedanz der Sensoreinheit 2, die von der Steuer- und Auswerteeinheit beispielsweise durch Auswertung von Betrag und Phase der Betriebsspannung ermittelt wird. Im vorliegenden Fall stellt die Sensoreinheit 2 einen Kondensator mit der Funktionsschicht als Dielektrikum dar. Durch Einwirkung der zu ermittelnden Gaskomponente auf die Funktionsschicht, beispielsweise durch Adsorption oder Absorption, tritt eine von der Konzentration der Gaskomponente abhängige Änderung der elektrischen Leitfähigkeit und/oder von der Dielektrizitätskonstante der Funktionsschicht ein, was sich in einer Änderung des Realteils und/oder des Imaginärteils der komplexen Impedanz der Sensoreinheit 2 bemerkbar macht. Durch Auswertung dieser Messgröße kann von der Steuer- und Auswerteeinheit daher die entsprechende Gaskomponente detektiert werden. Es versteht sich, dass die Selektivität und die Empfindlichkeit der Sensoreinheit durch die Wahl des Funktionsschichtmaterials, die Frequenz der Betriebsspannung und durch die Art der Messgrößenauswertung in geeigneter Weise beeinflusst werden können. Im Folgenden wird davon ausgegangen, dass die Sensoreinheit zur Erfassung der Abgaskomponente Ammoniak (NH3) ausgelegt ist und die Steuer- und Auswerteeinheit über eine Impedanzmessung ein mit der NH3-Konzentration im Abgas korrelierendes Sensorsignal erzeugt. Das in Fig. 2 dargestellte Diagramm verdeutlicht diesen Sachverhalt.

In dem in Fig. 2 dargestellten Diagramm ist das von der Steuer- und Auswerteeinheit aus der Impedanz als Messgröße erzeugte Sensorsignal in Abhängigkeit von der Betriebsdauer aufgetragen. Dabei ist die Betriebsdauer in logarithmischem Maßstab auf der Abszisse abgetragen. Da das Sensorsignal aus der Messgröße resultiert, wird nachfolgend vereinfachend vom Sensorsignal gesprochen, wenn der Bezug zu der das Sensorsignal erzeugenden Messgröße klar ist. Zur Erzeugung des Sensorsignals wurde der Abgassensor 1 bzw. die Sensoreinheit 2 einem Abgas ausgesetzt, das einen sich stufig nach einem vorgegebenen zeitlichen Raster zwischen 0 ppm und 100 ppm ändernden Ammoniakgehalt aufwies. Infolgedessen wurde ein im Diagramm der Fig. 2 aufgetragenes Sensorsignal erzeugt, das Werte zwischen einer oberen, der Konzentration von 0 ppm zugeordneten Grenze 21 und einer unteren, der Konzentration von 100 ppm zugeordneten Grenze 22 aufweist.

Aus dem Diagramm der Fig. 2 ist jedoch ersichtlich, dass eine mit der Betriebsdauer zunehmende Änderung der Sensoreigenschaften stattfindet, welche sich in einem Absinken sowohl der oberen Grenze 21 des Sensorsignals als auch der unteren Grenze 22 des Sensorsignals äußert. Zudem rücken die Grenzen 21, 22 mit zunehmender Betriebsdauer näher zusammen, was einer abnehmenden Sensorempfindlichkeit entspricht.

Erfindungsgemäß wird ein langzeitstabiles Sensorsignal dadurch erhalten, dass von der Steuer- und Auswerteeinheit eine Vorspannung 17 an den ersten Anschluss 4 und/oder an den zweiten Anschluss 5 der Sensoreinheit 2 angelegt wird. Die Vorspannung ist hierbei in Abhängigkeit von einer Sensoreigenschaft und/oder in Abhängigkeit von einer Sensorbelastung einstellbar, so dass über die Betriebsdauer ein langzeitstabiles Sensorverhalten beispielsweise hinsichtlich des Nullpunktssignals oder der Empfindlichkeit erzielt wird. Vorzugsweise weist die Vorspannung 17 eine positive Polung gegenüber einem in Bezug auf die Sensoreinheit 2 unter der Isolierschicht 3 angeordneten Schaltkreis, insbesondere gegenüber dem Schaltkreis 6, 8, 9 des Temperaturfühlers auf und wird an beide Anschlüsse 4, 5 der Sensoreinheit angelegt. Vorzugsweise ist der Temperaturfühlerschaltkreis 6, 8, 9 als Widerstandsthermometer ausgeführt, der an eine Konstantstromquelle der Steuer- und Auswerteeinheit angeschlossen ist. Die Betriebsspannung des Widerstandsthermometers in Bezug auf das gemeinsame Massepotential 18 liegt in diesem Fall im Millivoltbereich. Somit ist das Potential der entsprechenden Anschlüsse 8, 9 vergleichsweise gering und es kann eine in Bezug auf diese Potentiale geringe Vorspannung 17 zur Erzielung der gewünschten Sensorstabilität ausreichen. Vorzugsweise liegt die Höhe der Vorspannung im Bereich zwischen 0,1 V und 25 V, besonders bevorzugt im Bereich zwischen 1,5 V und 3,5 V.

Falls die Trennschicht 10 ebenfalls an eine Betriebsspannung angeschlossen ist, so ist es vorteilhaft, die Polung der Vorspannung 17 auch in Bezug auf das Potential der Trennschicht 10 positiv zu wählen.

Die Höhe der Vorspannung 17 kann sich an einem Referenzwert des Sensorsignals, beispielsweise dem Nullpunktswert des Sensorsignals orientieren. Hierzu ist es vorteilhaft, bei Betriebspunkten, bei denen sichergestellt ist, dass der Ammoniakgehalt des Abgases Null oder vernachlässigbar klein ist, die Vorspannung 17 kontinuierlich oder in Schritten solange zu verändern, bis das Sensorsignal den vorgegebenen Nullpunktswert annimmt. Diese justierende Einstellung kann bei jedem n-ten Einschaltvorgang des Abgassensors 1 vorgenommen werden. Vorteilhaft ist es, wenn sie bei jedem Einschaltvorgang des Abgassensors 1 vorgenommen wird und die eingestellte Vorspannung 17 bis zum Abschalten des Abgassensors 1 angelegt bleibt. Analog kann bei einem Betriebspunkt verfahren werden, bei welchem eine bekannte Ammoniakkonzentration im Abgas als Referenzwert vorliegt.

Es kann auch vorgesehen sein, dass die Höhe der Vorspannung 17 in Abhängigkeit von der Empfindlichkeit der Sensoreinheit 2 eingestellt wird. Hierzu ist es zweckmäßig, wenn bei Vorliegen einer bekannten Ammoniakkonzentration die Vorspannung 17 kontinuierlich oder in Schritten solange geändert wird, bis das Sensorsignal den, beispielsweise in Form einer Sollkennlinie vorgegebenen, Sollwert für die jeweilige Ammoniakkonzentration annimmt. Ebenfalls vorteilhaft ist es, eine in Bezug auf die zu detektierende Gaskomponente NH3 gegebenenfalls vorhandene Querempfindlichkeit des Abgassensors 1 gegenüber einer weiteren Abgaskomponente, wie beispielsweise Wasser (H2O) oder Kohlenmonoxid (CO) zur Einstellung der Vorspannung 17 auszunutzen. Dabei wird die Erkenntnis ausgenutzt, dass eine Drift der Sensorempfindlichkeit gegenüber NH3 im allgemeinen annähernd parallel zu einer Drift der Sensorquerempfindlichkeiten verläuft. Da die H2O- bzw. die CO-Konzentration im Abgas anhand der Motorbetriebsbedingungen ermittelt werden kann, kann insbesondere bei einer vernachlässigbaren NH3-Konzentration die Justierung der Vorspannung 17 dadurch vorgenommen werden, dass bei bekannten H2O- bzw. CO-Konzentrationen im Abgas das Sensorsignal durch Veränderung der Vorspannung 17 auf diesen Konzentrationen zugeordnete Werte gebracht wird. Besonders vorteilhaft ist in diesem Zusammenhang, vorbestimmte Werte für die Konzentration einer Gaskomponente, gegenüber der eine Querempfindlichkeit vorliegt, als Referenzwerte bestimmten Motorbetriebsbedingungen zuzuordnen und die Einstellung der Vorspannung 17 in einer Art Kalibrierung vorzunehmen, wenn ein entsprechender Motorbetriebszustand vorliegt. Gegebenenfalls können abgespeicherte Werte für das Sensorsignal bei einer oder mehreren vorangegangenen Messungen hierzu mit herangezogen werden.

Es kann ferner zweckmäßig sein, die Höhe der Vorspannung 17 in Abhängigkeit von einer Sensoreigenschaft einzustellen, welche sich parallel zu der beschriebenen Drift des Sensorsignals ändert. Ein geeignete Sensoreigenschaft ist beispielsweise eine zwischen der Elektrodenstruktur 20 und dem Schaltkreis 11, 13, 14 der Heizung bzw. dem Schaltkreis 6, 8, 9 des Temperaturfühlers messbare Bezugsgröße, wie insbesondere die elektrische Leitfähigkeit oder die Kapazität. Die zugrunde liegende Relation kann als Tabelle oder als funktionaler Zusammenhang in der Steuer- und Auswerteeinheit abgespeichert sein. Von Zeit zu Zeit oder in vorgegebenen regelmäßigen Abständen wird von der Steuer- und Auswerteeinheit beispielsweise die Kapazität oder die Leitfähigkeit zwischen der Elektrodenstruktur 20 und dem Temperaturfühler 6 ermittelt und die Vorspannung entsprechend des abgespeicherten Zusammenhangs eingestellt.

Ferner kann es vorgesehen sein, den Einfluss der Sensorbelastung auf die Stabilität des Sensorsignals zu ermitteln und die Vorspannung in Abhängigkeit von der Sensorbelastung einzustellen. Zur Kennzeichnung der Sensorbelastung kann beispielsweise das Produkt aus Ammoniakkonzentration und Messzeit bei der entsprechenden Ammoniakkonzentration (ppm * h) herangezogen werden. Ebenfalls zweckmäßig ist es, das Produkt aus Abgastemperatur und Betriebszeit heranzuziehen. Gegebenenfalls können zusätzlich lineare oder nichtlineare Korrekturfaktoren mit berücksichtigt werden. Im einfachsten Fall kann die Betriebsdauer als Maß für die Sensorbelastung dienen und die Vorspannung auf der Basis einer der Steuer- und Auswerteeinheit zur Verfügung stehenden vorgegebenen Abhängigkeit von der Betriebsdauer eingestellt werden. Auf die beschriebene Weise ist es möglich, über lange Zeit ein zuverlässiges und stabiles Messverhalten des Abgassensors 1 bzw. des Sensorsignals zu erzielen.

Es versteht sich, dass die genannten Maßnahmen auch bei modifizierten Ausführungsformen von Abgassensoren angewendet werden können.

## Patentansprüche

1. Abgassensor (1) zur Detektion einer Gaskomponente im Abgas einer Brennkraftmaschine, mit einer Steuer- und Auswerteeinheit und einer Sensoreinheit (2) mit einer Elektrodenstruktur (20) mit einem ersten Anschluss (4) und einem zweiten Anschluss (5), wobei der Steuer- und Auswerteeinheit zur Ermittlung der Konzentration der Gaskomponente eine zwischen dem ersten Anschluss (4) und dem zweiten Anschluss (5) der Elektrodenstruktur (20) vorhandene elektrische Messgröße zuführbar ist,
**dadurch gekennzeichnet, dass** die Steuer-und Auswerteeinheit so eingerichtet ist, dass sie
aus der Messgröße eine Impedanz der Sensoreinheit (2) ermittelt, und eine gegenüber einem Massepotential (18) definierte Vorspannung (17) an den ersten Anschluss (4) und/oder an den zweiten Anschluss (5) der Elektrodenstruktur (20) anlegt, wobei sie die Höhe der Vorspannung (17) in Abhängigkeit von einer Sensoreigenschaft und/oder in Abhängigkeit von einer Sensorbelastung derart einstellt, dass über die Betriebsdauer ein langzeitstabiles Sensorverhalten hinsichtlich eines Nullpunktssignals und/oder einer Empfindlichkeit erzielt wird.

2. Gassensor nach Anspruch 1,
**dadurch gekennzeichnet, dass**
die Höhe der Vorspannung (17) in Abhängigkeit von einem Referenzwert der Messgröße eingestellt wird.

3. Gassensor nach Anspruch 1 oder 2,
**dadurch gekennzeichnet, dass**
die Höhe der Vorspannung (17) in Abhängigkeit von der Empfindlichkeit der Sensoreinheit (2) eingestellt wird.

4. Gassensor nach einem der vorangehenden Ansprüche,
**dadurch gekennzeichnet, dass**
die Höhe der Vorspannung in Abhängigkeit von einer zwischen der Elektrodenstruktur (20) der Sensoreinheit und einem Schaltkreis des Abgassensors (1) messbaren elektrischen Bezugsgröße eingestellt wird.

5. Gassensor nach Anspruch 4,
**dadurch gekennzeichnet, dass**
der Gassensor (1) einen mit einer Isolierschicht (3) abgedeckten Schaltkreis (6, 8, 9) zur Temperaturmessung aufweist, wobei die Sensoreinheit (2) auf der Isolierschicht (3) aufgebracht ist und die Höhe der Vorspannung (17) in Abhängigkeit von einer zwischen der Elektrodenstruktur (20) der Sensoreinheit (2) und dem Schaltkreis (6, 8, 9) zur Temperaturmessung messbaren elektrischen Bezugsgröße eingestellt wird.

6. Gassensor nach Anspruch 1,
**dadurch gekennzeichnet, dass**
die Höhe der Vorspannung (17) in Abhängigkeit von der Betriebsdauer des Gassensors (1) eingestellt wird.

7. Gassensor nach einem der vorangehenden Ansprüche,
**dadurch gekennzeichnet,**
**dass** die Vorspannung (17) eine positive Polung in Bezug auf eine Betriebsspannung eines Schaltkreises des Abgassensors aufweist.

8. Gassensor nach einem der vorangehenden Ansprüche,
**dadurch gekennzeichnet,**
**dass** der Abgassensor (1) zur Erfassung der Gaskomponente Ammoniak ausgelegt ist.

9. Verfahren zum Betreiben eines Abgassensors (1) zur Ermittlung der Konzentration einer Gaskomponente im Abgas einer Brennkraftmaschine, wobei der Abgassensor (1) eine gassensitive Sensoreinheit (2) mit einer Elektrodenstruktur (20) mit einem ersten Anschluss (4) und mit einem zweiten Anschluss (5) aufweist, und zwischen dem ersten Anschluss (4) und dem zweiten Anschluss (5) der Elektrodenstruktur (20) eine mit der Konzentration der Gaskomponente korrelierende elektrische Messgröße abgegriffen wird,
**dadurch gekennzeichnet, dass** aus der Messgröße eine Impedenz der Sensoreinheit (2) ermittelt wird, und dass eine gegenüber einem Massepotential (18) definierte
Vorspannung (17) an den ersten Anschluss (4) und/oder an den zweiten Anschluss (5) der Elektrodenstruktur (20) angelegt wird, wobei die Höhe der Vorspannung (17) in Abhängigkeit von einer Sensoreigenschaft und/oder in Abhängigkeit von einer Sensorbelastung derart eingestellt wird, dass über die Betriebsdauer ein langzeitstabiles Sensorverhalten hinsichtlich eines Nullpunktssignals und/oder einer Empfindlichkeit erzielt wird.

10. Verfahren nach Anspruch 9,
**dadurch gekennzeichnet,**
**dass** die Höhe der Vorspannung (17) in Abhängigkeit von der Nullpunktsdrift der elektrischen Messgröße eingestellt wird.

11. Verfahren nach Anspruch 9 oder 10,
**dadurch gekennzeichnet,**
**dass** die Höhe der Vorspannung (17) in Abhängigkeit von einer Empfindlichkeitsdrift des Abgassensors (1) eingestellt wird.

12. Verfahren nach einem der Ansprüche 9 bis 11,
**dadurch gekennzeichnet,**
**dass** die Höhe der Vorspannung (17) zu vorgebbaren Zeitpunkten eingestellt wird.

13. Verfahren nach einem der Ansprüche 9 bis 12,
**dadurch gekennzeichnet,**
**dass** die Höhe der Vorspannung (17) bei jedem n-ten Einschaltvorgang des Abgassensors (1) eingestellt wird.

14. Verfahren nach einem der Ansprüche 9 bis 12,
**dadurch gekennzeichnet,**
**dass** die Vorspannung (17) positiv in Bezug auf eine Betriebsspannung eines elektrisch von der Sensoreinheit 2 isolierten Schaltkreises des Abgassensors (1) eingestellt wird.

## Claims

1. Exhaust gas sensor (1) for the detection of a gas component in the exhaust gas from an internal combustion engine, with a control and evaluation unit and a sensor unit (2) having an electrode structure (20) with a first terminal (4) and a second terminal (5), such that to determine the concentration of the gas component a measured electric parameter existing between the said first (4) and second (5) terminals of the electrode structure (20) can be fed to the control and evaluation unit,
**characterised in that** the control and evaluation unit is designed to determine from the said measured parameter an impedance of the sensor unit (2), and a polarising potential (17) defined relative to an earth potential (18) is applied to the first terminal (4) and/or to the second terminal (5) of the electrode structure (20), the level of the said polarising potential (17) being set to a value as a function of a sensor property and/or as a function of a sensor load, such that over the operating time of the sensor lastingly stable sensor behaviour is achieved in relation to a zero-point signal and/or its sensitivity.

2. Gas sensor according to Claim 1,
**characterised in that**
the level of the polarising potential (17) is set as a function of a reference value of the measured parameter.

3. Gas sensor according to Claims 1 or 2,
**characterised in that**
the level of the polarising potential (17) is set as a function of the sensitivity of the sensor unit (2).

4. Gas sensor according to any of the preceding claims,
**characterised in that**
the level of the polarising potential (17) is set as a function of a reference parameter that can be measured between the electrode structure (20) of the sensor unit and a switching circuit of the exhaust gas sensor (1).

5. Gas sensor according to Claim 4,
**characterised in that**
the gas sensor (1) comprises a switching circuit (6, 8, 9) for temperature measurement covered by an insulating layer (3), such that the sensor unit (2) is attached on the insulating layer (3), and the level of the polarising potential (17) is set as a function of a measurable electric reference magnitude between the electrode structure (20) of the sensor unit (2) and the switching circuit (6, 8, 9) for temperature measurement.

6. Gas sensor according to Claim 1,
**characterised in that**
the level of the polarising potential (17) is set as a function of the operating time of the gas sensor (1).

7. Gas sensor according to any of the preceding Claims,
**characterised in that**
the polarising potential (17) has positive polarity relative to an operating voltage of a switching circuit of the exhaust gas sensor.

8. Gas sensor according to any of the preceding claims,
**characterised in that**
the exhaust gas sensor (1) is designed to detect the gas component ammonia.

9. Method for the operation of an exhaust gas sensor (1) for determining the concentration of a gas component in the exhaust gas from an internal combustion engine, such that the exhaust gas sensor (1) comprises a gas-sensitive sensor unit (2) having an electrode structure (20) with a first terminal (4) and a second terminal (5), and such that a measured electric parameter which correlates with the concentration of the gas component is determined between the said first (4) and second (5) terminals of the said electrode structure (20),
**characterised in that**
from the said measured parameter an impedance of the sensor unit (2) is determined, and a polarisation potential (17) defined relative to an earth potential (18) is applied to the first terminal (4) and/or to the second terminal (5) of the electrode structure (20), the level of the said polarising potential (17) being set to a value as a function of a sensor property and/or as a function of a sensor load, such that over the operating time of the sensor lastingly stable sensor behaviour is achieved in relation to a zero-point signal and/or its sensitivity.

10. Method according to Claim 9,
**characterised in that**
the level of the polarising potential (17) is set as a function of the zero-point drift of the measured electric parameter.

11. Method according to Claims 9 or 10,
**characterised in that**
the level of the polarising potential (17) is set as a function of the sensitivity drift of the exhaust gas sensor (1).

12. Method according to Claims 9 or 11,
**characterised in that**
the level of the polarising potential (17) is adjusted at specifiable points in time.

13. Method according to Claims 9 or 12,
**characterised in that**
the level of the polarising potential (17) is set every n-th time that the exhaust gas sensor (1) is switched on.

14. Method according to Claims 9 or 13,
**characterised in that**
the level of the polarising potential (17) is set to be positive relative to an operating voltage of a switching circuit of the exhaust gas sensor (1) which is electrically insulated from the sensor unit (2).

## Revendications

1. Capteur de gaz d'échappement (1) pour la détection d'un composant gazeux dans les gaz d'échappement d'un moteur à combustion interne, avec une unité de commande et d'analyse et une unité de capteur (2) présentant une structure d'électrode (20) avec une première connexion (4) et une deuxième connexion (5), une grandeur de mesure électrique présentée entre la première connexion (4) et la deuxième connexion (5) de structure d'électrode (20) étant applicable à l'unité de commande et d'analyse pour la détermination de la concentration du composant gazeux,
**caractérisé en ce que**
l'unité de commande et d'analyse est réglée de manière à déterminer une impédance de l'unité de capteur (2) à partir de la grandeur de mesure et à appliquer une tension de polarisation (17) définie relativement au potentiel de masse (18) à la première connexion (4) et/ou à la deuxième connexion (5) de la structure d'électrode (20), la valeur de la tension de polarisation (17) étant réglée par elle en fonction d'une propriété de capteur et/ou en fonction d'une contrainte du capteur de manière à obtenir un comportement de capteur stable à long terme pendant la durée de service relativement à un signal de point zéro et/ou à une sensibilité.

2. Capteur de gaz selon la revendication 1,
**caractérisé en ce que**
la valeur de la tension de polarisation (17) est réglée en fonction d'une valeur de référence de la grandeur de mesure.

3. Capteur de gaz selon la revendication 1 ou 2,
**caractérisé en ce que**
la valeur de la tension de polarisation (17) est réglée en fonction de la sensibilité de l'unité de capteur (2).

4. Capteur de gaz selon l'une des revendications précédentes,
**caractérisé en ce que**
la valeur de la tension de polarisation est réglée en fonction d'une grandeur électrique de référence mesurable entre la structure d'électrode (20) de l'unité de capteur et un circuit de commutation du capteur de gaz d'échappement (1).

5. Capteur de gaz selon la revendication 4,
**caractérisé en ce que**
le capteur de gaz (1) comporte un circuit de commutation (6, 8, 9) recouvert d'une couche isolante (3) pour la mesure de température, l'unité de capteur (2) étant appliquée sur la couche isolante (3) et la valeur de la tension de polarisation (17) étant réglée en fonction d'une grandeur électrique de référence mesurable entre la structure d'électrode (20) de l'unité de capteur (2) et le circuit de commutation (6, 8, 9) pour la mesure de température.

6. Capteur de gaz selon la revendication 1,
**caractérisé en ce que**
la valeur de la tension de polarisation (17) est réglée en fonction de la durée de service du capteur de gaz (1).

7. Capteur de gaz selon l'une des revendications précédentes,
**caractérisé en ce que**
la tension de polarisation (17) présente une polarité inverse relativement à une tension de service d'un circuit de commutation du capteur de gaz d'échappement.

8. Capteur de gaz selon l'une des revendications précédentes,
**caractérisé en ce que**
le capteur de gaz d'échappement (1) est conçu pour la saisie du composant gazeux qu'est l'ammoniac.

9. Procédé pour l'exploitation d'un capteur de gaz d'échappement (1) pour la détermination de la concentration d'un composant gazeux dans les gaz d'échappement d'un moteur à combustion interne, le capteur de gaz d'échappement (1) comprenant une unité de capteur (2) sensible aux gaz présentant une structure d'électrode (20) avec une première connexion (4) et une deuxième connexion (5), et une grandeur de mesure électrique corrélée à la concentration du composant gazeux étant saisie entre la première connexion (4) et la deuxième connexion (5) de la structure d'électrode (20),
**caractérisé en ce qu'**
une impédance de l'unité de capteur (2) est déterminée à partir de la grandeur de mesure, et **en ce qu'**une tension de polarisation (17) définie relativement au potentiel de masse (18) est appliquée à la première connexion (4) et/ou à la deuxième connexion (5) de la structure d'électrode (20), la valeur de la tension de polarisation (17) étant réglée en fonction d'une propriété de capteur et/ou en fonction d'une contrainte du capteur de manière à obtenir un comportement de capteur stable à long terme pendant la durée de service relativement à un signal de point zéro et/ou à une sensibilité.

10. Procédé selon la revendication 9,
**caractérisé en ce que**
la valeur de la tension de polarisation (17) est réglée en fonction de la dérive du zéro de la grandeur de mesure électrique.

11. Procédé selon la revendication 9 ou la revendication 10,
**caractérisé en ce que**
la valeur de la tension de polarisation (17) est réglée en fonction d'une dérive de sensibilité du capteur de gaz d'échappement (1).

12. Procédé selon l'une des revendications 9 à 11,
**caractérisé en ce que**
la valeur de la tension de polarisation (17) est réglée à des moments définissables.

13. Procédé selon l'une des revendications 9 à 12,
**caractérisé en ce que**
la valeur de la tension de polarisation (17) est réglée à chaque n^{ième} commutation du capteur de gaz d'échappement (1).

14. Procédé selon l'une des revendications 9 à 12,
**caractérisé en ce que**
la tension de polarisation (17) est réglée avec une polarité inverse relativement à une tension de service d'un circuit de commutation du capteur de gaz d'échappement (1) électriquement isolé de l'unité de capteur (2).
